# EUROPEAN PATENT SPECIFICATION

(11) **EP 0 256 676 B1**
(45) Date of publication and mention of the grant of the patent: **05.10.1994**
(21) Application number: 87306340.8
(22) Date of filing: 17.07.1987
(51) Int. Cl.: G01N 33/487, G01N 33/68

(54) **A continuous flow reactor for peptide sequenators**
Durchlaufreaktor für Peptidsequenatoren
Réacteur continu pour des séquenceurs peptidiques

(30) Priority: 15.08.1986 US 896724
(43) Date of publication of application: 24.02.1988
(73) Proprietor: Beckman Research Institute of the City of Hope, Duarte California 91010 (US)
(72) Inventor: Shively, John Ernest, Arcadia, CA 91006 (US)
(74) Representative: De Minvielle-Devaux, Ian Benedict Peter

(56) References cited:
- DE-A- 2 720 375
- GB-A- 2 084 899
- US-A- 4 483 964
- ANALYTICAL BIOCHEMISTRY, vol. 147, 1985, New York, NY (US); D.H. HAWKE et al., pp. 315-330#
- EUROPEAN JOURNAL OF BIOCHEMISTRY, vol. 20, no. 1, 1971, Berlin (DE); R.A. LAURSEN, pp. 89-102#
- ANAL. BIOCHEM., vol. 163, 1987; SHIVELY et al., pp. 517-529#

## Description

### Background of the Invention

### A. Field of the Invention

This invention relates to apparatus and methods for peptide sequencing. In particular, the invention relates to a continuous flow column reactor for a peptide sequenator and to a peptide sequencing method in which the reactor is utilised.

### B. Description of the Prior Art

Practical automated peptide sequencing dates from the 1967 introduction of the spinning cup sequenator. See Edman, P., and Begg, G., A Protein Sequenator, European Journal of Biochemistry, 1, 80-91 (1967). A focal problem associated with the spinning cup sequenator is sample loss, particularly of short peptides. Therefore, an alternative method-solid phase degradation-was designed whereby reagents and solvents are passed in an appropriate program through a column packed with a solid support such as polystyrene matrix or preferably glass beads to which a peptide is covalently attached. Both the column and the tubing by wheich it is connected to the sequenator may be formed from polytetrafluoroethylene, e.g., Teflon. See Laursen, R.A., A Solid-Phase Peptide Sequenator, European Journal of Biochemistry, 20, 89-102 (1971) and Shively, "Methods of Protein Mischaracterization," Humana Press, Clifton, N. J. (1986), Chapter 9.

In 1981, a sequenator that employs gas phase reagents instead of liquid phase reagents at critical points in the Edman degradation was proposed. See Hewick, R.M., Hunkapillar, M.W., Hood, L.E., Dreyer, W.J., A Gas-Liquid Solid Phase Peptide and Protein Sequenator, The Journal of Biological Chemistry, 256, 7990-7997 (1981) and Shively, supra, Chapter 8, Sec. 314, p. 229. This device includes a two-part glass cartridge reaction assembly which houses a miniature continuous flow glass reaction chamber in which the peptide sample is presented as a dispersion in a thin film of Polybrene supported on a porous glass fiber disk. Means are provided for disconnecting the cartridge from its mounting base each time the sample is loaded. The cartridge is vertically mounted in a sequenator to which it is connected at its inlet and outlet ends by Teflon tubing.

A modification of the Hewick, et al. sequenator is described by Hawke, Harris and Shively in Analytical Biochemistry, 147, 315-330 (1985), and Shively, supra, Chapter 7, page 210, et seq. This modification replaces the glass reactor cartridge assembly of Hewick, et al. with an all Teflon cartridge of similar design, thus providing an all Teflon delivery and reaction system. The sample is presented within the reaction chamber on a trimethylsilyated glass fiber disk. Hawke, et al., noting that Teflon is "self-sealing", report lower background levels deemed to be consequent from a better seal achieved in the all Teflon design as compared to the seal observed with the Hewick, et al. glass cartridge. The all Teflon design of the instrument is also reported to be responsible for increased yields. See Shively, supra, at p. 217.

Design of a multipurpose sequenator is discussed in Shively, supra, in Chapter 9, page 249, et seq. Such a multipurpose machine is constructed in units which are interchangeable so that it may be easily converted to run with a cup compartment, a column, or a cartridge. In the discussion of "Exchangeable Parts", it is explained the micro columns adapted for microsequencing in solid phase sequenators can be replaced by a cartridge without changing the other parts of the apparatus. A polyfluorochloro (Kel-F) micro column filled with peptide bound glass support and provided with Teflon tubing inlet and outlet lines for attachment to a sequenator is described.

GB-A-1 540 956 and DE-A-2 720 375 disclose a continuous flow reactor which comprises:
a reaction chamber to be packed with peptide coated discrete objects and formed from a tube; and
first and second tubes for passing reactive fluids and solvents from a peptide sequenator into the reaction chamber and for removal of solvents and reaction products from the reaction chamber respectively.

US-A-4483964 describes a tubular reactor which has application in different solid phase syntheses or degradations. The reactor consists of a glass column, filled with the desired solid phase material, linked to two flexible conduits made from chemically inert material. These conduits allow the column to communicate with the manifold.

### Summary of the Invention

This invention provides a continuous flow column reactor substantially free of unswept volumes in which accumulation of amino acids, by products and reagents is substantially eliminated. The reactor is inexpensive and is easily inserted into and removed from the sequenator, thus facilitating the frequent use of new and uncontaminated chambers.

This is achieved by a reactor according to Claim 1.

### Brief Description of the Drawings

The novel features of the invention will be more readily appreciated from the following description when read in conjunction with the appended drawings, in which corresponding components are designated by the same reference numerals throughout the various figures.
Figure 1 is an elevational view, in section, of a continuous flow reactor constituting one embodiment of the invention;
Figure 2 is a sectional view, in plan, of a discrete object with one coating used in the continuous flow reactor shown in Figure 1;
Figure 3 is a sectional view, in plan, of a discrete object, e.g., glass bead, with two coatings used in the continuous flow reactor shown in Figure 1;
Figure 4 is an elevational view, in section, of a continuous flow reactor constituting an alternative embodiment of the invention; and
Figure 5 is a comparative set of chromatograms obtained using a continuous flow reactor of the present invention and a prior art cartridge reactor chamber.

### Detailed Description of the Invention

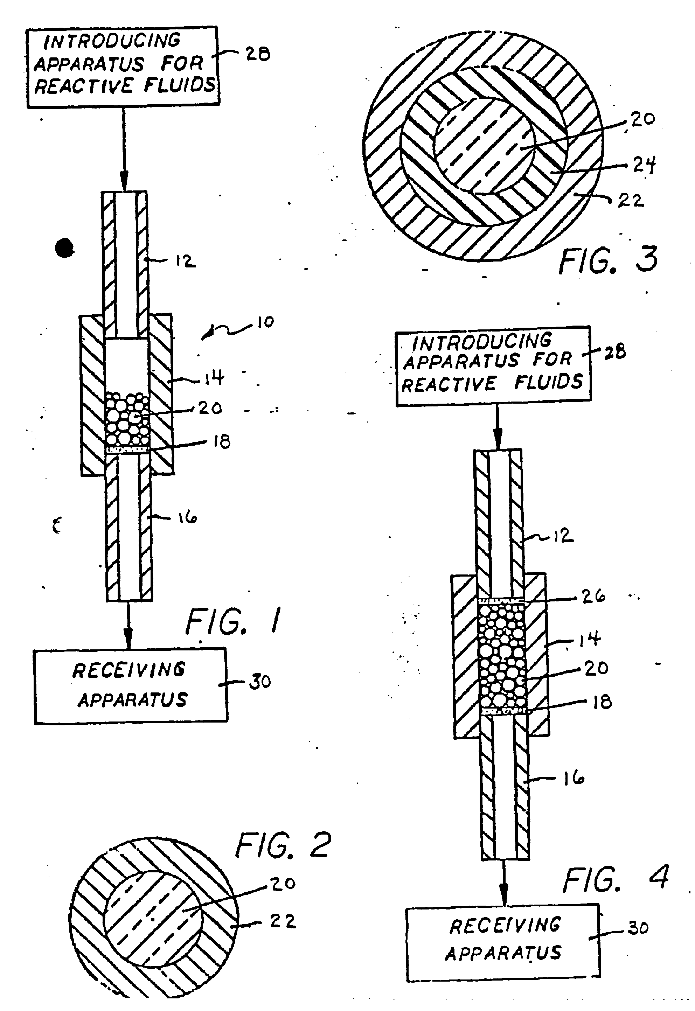

A continuous flow reactor according to the present invention is shown in Figure 1 where it is generally designated by reference numeral 10. The continuous flow reactor 10 includes reaction tube 14 and interference fitted supply and drain tubes 12 and 16. Each of the tubes 12, 14 and 16 is formed from chemically inert, pliable synthetic resinous material, preferably a self-lubricating fluorocarbon such as a polytetrafluorethylene. Numerous fluorocarbons are available. See, e.g., Plastics Engineering Handbook, Van Nostrand Reinhold Co. (1976), pp. 60-62.

Each of the tubes 12 and 16 may be of substantially the same size, and the reaction tube 14 may be larger than the tubes 12 and 16. For example, the tubes 12 and 16 may have an outer diameter of approximately one-sixteenth of an inch (1/16˝) and the reaction tube 14 may have an inner diameter of approximately one-sixteenth of an inch (1/16˝) and an outer diameter of approximately one-eighth of an inch (1/8˝). Preferably, the inner diameter of the reaction tube 14 is slightly undersized relative to the outer diameter of the tubes 12 and 16. With these size relationships, the tubes 12 and 16 can be interference or press fitted into the opposite ends of the reaction tube 14 to provide leak-tight joints between the tubes 12 and 16 and the reaction tube 14.

Alternatively, the tube 16 may be larger and so dimensioned as to provide a press fit on the outside instead of the inside of the reaction tube 14 as shown in Figure 1. A reaction zone free of unswept volumes is provided in this manner.

A porous support member 18, such as a disk, is tightly fitted inside the reaction tube 14. Preferably, the support member 18 is positioned near the location of the upper edge of the tube 16 in the embodiment shown in Figure 1. The support member 18 has a porosity requisite to allow passage of fluids and yet retain discrete objects 20, such as beads packed into the upper portion of the reaction tube 14. Support member 18 is formed from a chemically inert synthetic resin, preferably a fluorocarbon. For example, the support member 18 may be made from polytetrafluoroethylene cut by a 14-gauge needle to provide a circular disk slightly larger than the inner diameter of the reaction tube 14. The disk can be pressed into the reaction tube 14 where it will be retained in the desired position by the resulting press fit.

Useful support members 18 may be formed from a synthetic resinous material sold under the trademark "ZITEX". This material is available from a number of suppliers including Norton Chemplast, 150 Dey Road, Wayne, New Jersey in different porosities such as extra fine, fine and medium. Materials with all of these different porosities can be used satisfactorily in the continuous flow reactor 10 constituting this invention.

The reaction tube 14 is approximately packed with discrete objects 20. Preferably, the discrete objects are made from a material such as a porous silica. The discrete objects 20 may be irregular or spherical. Suitable discrete objects 20 having an irregular and porous configuration may be obtained from Electro-Nucleonics, 368 Passaic Avenue, Fairfield, New Jersey. Preferred irregular discrete objects 20 have a size between 74 to 125 µm (200 - 120 mesh size) and a pore size of approximately 37,9 nm (379 A°). Silica packing material meeting these specifications are available as GC Porasils B and C from Waters Chromatography Division of Millipore Corporation, 34 Maple Street, Milford, Massachusetts. See Waters Sourcebook for Chromatography Columns and Supplies (1986).

Spherical discrete objects 20 having a diameter of from about one hundred microns (100µm) to three hundred microns (300µm) are preferred. Each spherical object when packed in the reaction chamber 14 provides spacings which assure that fluids will flow along substantially non-linear paths and will be drained without entrapment. The provision of non-linear flow paths is further faciliated by the use of packings which comprise a plurality of different sized spherical objects. For example, a mixture of spherical particles of different diameters in the range of one hundred microns (100µm) to three hundred microns (300µm) is appropriate.

Silica derivatives, such as octadecyl silica and octyl silica, may also be used for the discrete objects 20 and may be preferred for the sequencing of certain peptides or proteins. Various other silica derivatives currently available for reverse phase high performance liquid chromatography may be used. Such derivatives may be either specifically prepared for use in the reactor of this invention, manufactured or purchased. Waters GC Porasils B and C and Waters GC Bondapack C18 material have been used as a starting material for the preparation of silica derivatives.

The discrete objects 20 can be coated with a peptide 22 (see Figure 2) which is to be sequenced. For example, one microgram of the peptide 22 may be provided for each milligram of the discrete objects 20. In a specific case, 1-3 micrograms of sperm whale apomyoglobin have been added to 5-10 milligrams of the discrete objects 20.

The minimum amount of sample depends on the purity, molecular size and the desired number of amino acid residues to be determined and can be as low as 0.1 to 10 picomoles. 10 to 20 milligrams of discrete objects 20 is frequently adequate to retain sample amounts in the range of 0.1 to 10,000 picomoles. However, the required quantity of such objects may vary by as much as much as tenfold for certain applications.

Alternatively, a first coating material 24 (see Figure 3), such as Polybrene which has affinity both for the objects and the peptides, may be coated on the discrete objects 20 before the peptide 22 is applied. A Polybrene coating is particularly appropriate when porous discrete objects are utilized. Preferably, at least one milligram of Polybrene per milligram of discrete objects 20 is applied. However, the amount of Polybrene may vary above or below this amount by approximately fifty percent (50%).

When Polybrene is applied to 5-10 mg of the discrete objects 20 which are packed in the reaction tube 14 with a length of 3 cm and a particle bed of 0.5-1.0 cm (5-10 mg of silica), an amount of approximately five microliters (5µl) of 100 mg/ml Polybrene may be applied to the reaction tube 14 so there is an adequate filling with the discrete objects 20 to a height of approximately 0.5-1.0 cm. When applied to the discrete objects 20 on a bulk basis outside of the reaction tube 14, a volume of 100 mg/ml Polybrene sufficient to wet the discrete objects 20 over their complete surfaces is applied. For 10 mg of silica, a solution of approximately 300 mg/ml of Polybrene may be employed. The discrete objects 20 may then be dried in a vacuum desicator.

In an alternative embodiment of the invention, a closure member 26 (see Figure 4) is disposed in the reaction tube 14 at a position near the end of the tube 12. The closure member 26 may be constructed in a manner similar to that for the support member 18. The closure member 26 encloses the top of the reaction tube 14 to confine the discrete objects 20.

The continuous flow reactor 10 is connected by the tube 12 to an apparatus (sequenator) for introducing reactive fluids, e.g., Edman reagents, for N-terminal sequencing, or Stark reagents for C-terminal sequencing.

The continuous flow reactor 10 described above has certain important advantages. These include the following:
(1) It is inexpensive, disposable, simply to construct, and easy to install into and remove from a sequenator. These features permit a series of reactors to be precharged and subsequently inserted into the sequenator as needed.
(2) It has little unwept volume, i.e., volumes not flushed with fluids, where materials can accumulate. Substantially, leakproof seals to vapors and fluids are provided throughout the length of the reactor. These features minimize the accumulation of by-products or successive amino acids isolated from the sequenced peptide and, hence, the background which may interfere with the chromatogram identification of successive amino acids from the peptides.
(3) It has few, if any, surfaces at which amino acids or by-products can be entrapped and accumulated which would later leach back into the flow stream and generate background signals in successive isolations of amino acids derivatives from a peptide.
(4) The discrete objects 20 disposed in the reaction tube 14 can be used as a sample concentrator in a method similar to that used in reverse phase high performance liquid chromatography technology. The continuous flow reactor 10 is compatible with high performance liquid chromatography technology and Edman and Stark chemistry technology. The discrete objects 20 in the continuous flow reactor 10 provide for good mass transfer characteristics which are clearly superior to the characteristics in existing cartridge technology

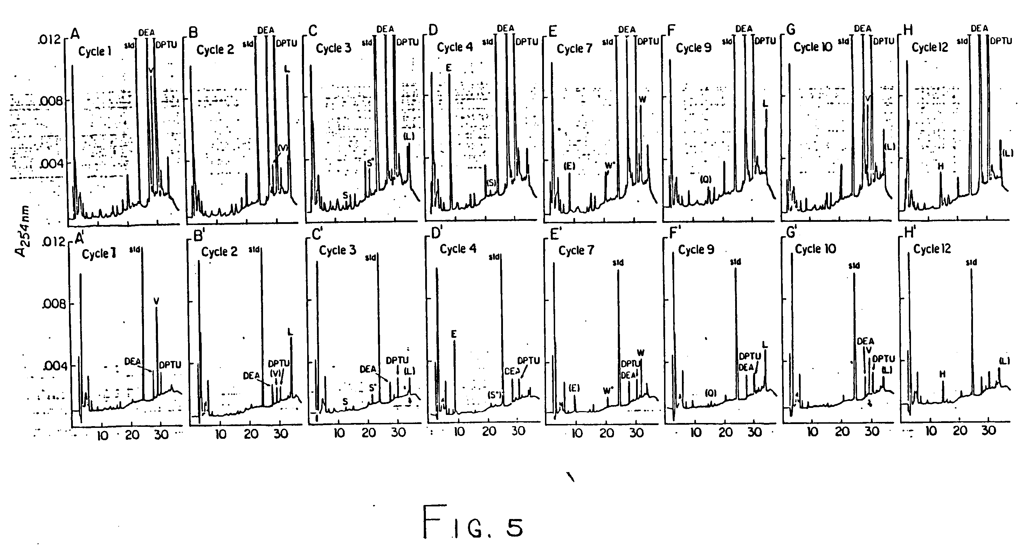

Figure 5 shows chromatograms which provide comparisons of sequencing results, obtained by using sperm whale apomyoglobin, between a cartridge reaction chamber as shown in Hawke, et al., supra, and a continuous flow reactor 10 of this invention. Specifically, figure 5 shows comparative sequencing results obtained in cycle 1 through cycle 4, and the results obtained in cycles 7, 9, 10 and 12.

The upper panels A-H in Figure 5 show selected Edman degradation cycles for the sequencing of 200 picomoles of sperm whale apomyoglobin. The sample was sequenced using a cartridge reaction chamber made from polytetrafluoroethylene as described in the manuscript by Hawke et al (1985) cited above. Before the sample could be applied to the glass fiber disk in the cartridge reaction chamber, it was necessary to coat the glass fiber disk with Polybrene (1 mg) and precycle the disk for two cycles of Edman chemistry. Since the time required for one cycle of Edman chemistry (removal of one amino acid derivative) is about 45 minutes, the precycling adds 90 minutes to the analysis time.

The amino acid derivatives obtained from the cartridge reaction chamber were analysed by reverse phase high performance chromatography by a method similar to the method described in the above cited Hawke et al (1985).

The lower panels, A′-H′ in Figure 5 are the chromatograms obtained from an 80 picomole run of sperm whale apomyoglobin using a continuous flow reactor 10 as described in this patent. The continuous flow reactor 10 is connected to the same sequencing apparatus and analyzed in an identical way at the same attenuation settings as described above for the cartridge reaction chamber of the prior art.

The large offscale peaks labeled DEA and DPTU in the panels of Figure 5 are common background peaks observed in Edman chemistry. The DEA is the phenylthiocarbamyl derivative of diethylamine (DEA). DEA is a trace contaminant of triethylamine (TEA) used as a base in Edman chemistry. The DPTU peak is diphenylthiourea, which is formed from the reaction of phenylisothiocyanate (PITC) with aniline. Aniline is in turn formed from the base catalyzed destruction of PITC.

The peaks described in the previous paragraph plus a number of smaller, unidentified peaks, constitute the background noise which interferes with the identification of the phenylthiohydantoin (PTH) amino acid derivatives. In each cycle, the single letter labeled peak corresponds to the correct assignment: V - valine, L - leucine, S - serine (S′ - a breakdown product of serine), E - glutamic acid, W - tryptophan, and H - histidine. The peak labeled "std" is an internal standard (the PTH derivative of aminoisobutyric acid). The peaks labeled in parentheses in the panels are the carryover signals from the previous cycle. Its appearance on a chromatogram is normal.

Even though less than 50% of the amount of sperm whale apomyoglobin was sequenced on the continuous flow reactor 10, there is adequate sensitivity compared to the results obtained with the cartridge reaction chamber. This is seen, for example, by comparing the signals for valine (V) in panels A and A′ or the signals for leucine (L) in panels B and B′. Beyond the substantial sensitivity provided by the continuous flow reactor 10 of the present invention there is also an increased signal-to-noise ratio improvement provided by the continuous flow reactor 10. The improved signal-to-noise ratios can be directly observed by, for example, comparing the relationships of the signal magnitudes for DEA, V and DPTU in panels A and A′. In panel A the magnitudes of signals for DEA and DPTU, which constitute background noise signals, are greater than that for V, the sought-after assignment. This relationship of signal magnitudes is reversed in panel A′ where the results using the continuous flow reactor 10 are shown. Such improved signal-to-noise ratios are repeated in all of the panels.

The continuous flow reactor 10 of this invention considerably improved the capability of the sequencing apparatus to sequence reduced amounts of sample. In addition, the sample was directly analyzed after the addition of Polybrene. No precycling was required. This represents a savings in the time required before sample analysis can be started.

The above discussion and related illustrations of the present invention are directed primarily to preferred embodiments and practices of the invention.

## Claims

1. A continuous flow reactor (10) which comprises:
a reaction chamber (14) to be packed with peptide coated discrete objects (20) and formed from a tube; and
first (12) and second (16) tubes for passing reactive fluids and solvents from a peptide sequenator into the reaction chamber (14) and for removal of solvents and reaction products from the reaction chamber (14) respectively;
said first (12) and second (16) tubes being formed from a pliable, chemically inert material;
characterised in that the reaction chamber (14) is formed from a pliable, chemically inert material and that
the inside and outside diameters of the reaction chamber tube (14) and the first (12) and second (16) connecting tubes are so dimensioned that two leak-tight interference fit joints are provided by inserting one end section of a tube (12; 16) into the end section of another tube (14), one of the leak-tight interference fit joints being provided between the first tube (12) and the reaction chamber (14) and the other being provided between the second tube (16) and the reaction chamber (14).

2. A continuous flow reactor (10) as defined in Claim 1 in which the reaction chamber tube (14) and each of the first (12) and second (16) connecting tubes are formed from fluorocarbon polymer.

3. A continuous flow reactor (10) as defined in Claim 1 in which the reaction chamber tube (14) and each of the first (12) and second (16) connecting tubes are formed from polytetrafluoroethylene.

4. The continuous flow reactor (10) as defined in Claim 1 in which the reaction chamber is packed with peptide coated porous silica objects (20).

5. The continuous flow reactor (10) as defined in Claim 4 wherein said objects (20) are peptide coated porous silica beads.

6. The continuous flow reactor (10) as defined in Claim 1 in which the reaction chamber tube (14) is provided with a porous support means (18) for the discrete articles (20) with which the chamber is to be packed.

7. The continuous flow reactor (10) as defined in Claim 1 in which the inside and outside diameters of said reaction chamber tube (14) and said first (12) and second (16) connecting tubes are so dimensioned that said first (12) and second (16) connecting tubes are interference fitted inside the reaction chamber tube (14).

8. The continuous flow reactor (10) as defined in Claim 1 in which the inside and outside diameters of the first connecting tube (12) and the reactor chamber tube (14) are so dimensioned that the first connecting tube (12) is interference fitted inside the reaction chamber (14) and in which the inside and outside diameters of the reaction chamber tube (14) and the second connecting tube (16) are so dimensioned that the reaction chamber tube (14) is interference fitted inside the second connecting tube (16) to provide a reaction chamber free of unswept volumes.

## Patentansprüche

1. Durchflußreaktor (10) welcher beinhaltet:
eine Reaktionskammer (14), die mit Peptid-beschichteten einzelnen Objekten (20) gepackt wird und aus einem Rohr gebildet ist; und
ein erstes (12) und zweites (16) Rohr jeweils zum Leiten von reaktiven Fluiden und Lösungsmitteln von einem Peptid-Sequenator in die Reaktionskammer (14) bzw. zum Entfernen von Lösungsmitteln und Reaktionsprodukten von der Reaktionskammer (14);
wobei das erste (12) und zweite (16) Rohr aus einem biegsamen, chemisch inerten Material geformt ist;
dadurch gekennzeichnet, daß die Reaktionskammer (14) aus einem biegsamen, chemisch inerten Material geformt ist und daß
der Innen- und Außendurchmesser des Reaktionskammerrohres (14) und des ersten (12) und zweiten (16) Verbindungsrohrs so dimensioniert sind, daß zwei leckdichte preßgepaßte Verbindungen bereitgestellt sind, durch Einsetzen eines Endabschnittes eines Rohres (12; 16) in den Endabschnitt eines anderen Rohres (14), wobei eine der leckdichten preßgepaßten Verbindungen zwischen dem ersten Rohr (12) und der Reaktionskammer (14) und die andere zwischen dem zweiten Rohr (16) und der Reaktionskammer (14) bereitgestellt ist.

2. Durchflußreaktor (10) gemäß Anspruch 1, wobei das Reaktionskammerrohr (14) und das erste (12) und das zweite (16) Verbindungsrohr aus Fluorkarbon-Polymer gebildet sind.

3. Durchflußreaktor (10) gemäß Anspruch 1, wobei das Reaktionskammerrohr (14) und sowohl das erste (12) als auch das zweite (16) Verbindungsrohr aus Polytetrafluorethylen gebildet sind.

4. Durchflußreaktor (10) gemäß Anspruch 1, wobei die Reaktionskammer mit Peptid-beschichteten porösen Siliciumdioxidobjekten (20) gepackt ist.

5. Durchflußreaktor (10) gemäß Anspruch 4, wobei die Objekte (20) Peptid-beschichtete poröse Siliciumdioxidkugeln sind.

6. Durchflußreaktor (10) gemäß Anspruch 1, wobei das Reaktionskammerrohr (14) mit einer porösen Trageeinrichtung (18) für die einzelnen Objekte (20) versehen ist, mit der die Kammer gepackt werden soll.

7. Durchflußreaktor (10) gemäß Anspruch 1, wobei der Innen- und Außendurchmesser des Reaktionskammerrohrs (14) und des ersten (12) und des zweiten (16) Verbindungsrohrs so dimensioniert sind, daß das erste (12) und das zweite (16) Verbindungsrohr preßgepaßt innerhalb des Reaktionskammerrohrs (14) sind.

8. Durchflußreaktor (10) gemäß Anspruch 1, wobei der Innen- und Außendurchmesser des ersten Verbindungsrohres (12) und des Reaktorkammerrohres (14) so dimensioniert sind, daß das erste Verbindungsrohr (12) innerhalb der Reaktionskammer (14) preßgepaßt ist und wobei der Innen- und Außendurchmesser des Reaktionskammerrohres (14) und des zweiten Verbindungsrohres (16) so dimensioniert sind, daß das Reaktionskammerrohr (14) innerhalb des zweiten Verbindungsrohres (16) preßgepaßt ist, um eine Reaktionskammer bereitzustellen, die frei von undurchflossenen Volumina ist.

## Revendications

1. Réacteur à flux continu (10) qui comprend :
une chambre de réaction (14) destinée à être garnie d'articles discrets recouverts de peptides (20) et prenant la forme d'un tube, et
un premier tube (12) et un second tube (16) destinés à envoyer des fluides réactifs ainsi que des solvants provenant d'un séquenceur peptidique dans la chambre de réaction (14) et à enlever les solvants et produits de réaction provenant respectivement de la chambre de réaction (14),
lesdits premier tube (12) et second tube (16) étant réalisés à partir d'un matériau souple et chimiquement inerte,
caractérisé en ce que la chambre de réaction (14) est réalisée à partir d'un matériau souple et chimiquement inerte et en ce que
les diamètres interne et externe du tube de la chambre de réaction (14) ainsi que du premier tube (12) et du second tube (16) de raccordement possèdent des dimensions telles que deux joints à ajustement serré étanches aux fuites sont disposés par insertion d'une section d'extrémité d'un tube (12, 16) dans la section d'extrémité d'un autre tube (14), l'un des joints à ajustement serré étanches aux fuites étant disposé entre le premier tube (12) et la chambre de réaction (14) et l'autre étant disposé entre le second tube (16) et la chambre de réaction (14).

2. Réacteur à flux continu (10) selon la revendication 1, dans lequel le tube de chambre de réaction (14), de même que chacun des premier tube (12) et second tube (16) de raccordement, sont réalisés à partir d'un polymère fluorocarboné.

3. Réacteur à flux continu (10) selon la revendication 1, dans lequel le tube de chambre de réaction (14), de même que chacun des premier tube (12) et second tube (16) de raccordement, sont réalisés à partir de polytétrafluoroéthylène.

4. Réacteur à flux continu (10) selon la revendication 1, dans lequel la chambre de réaction est garnie avec des articles en silice poreuse recouverts de peptides (20).

5. Réacteur à flux continu (10) selon la revendication 4, dans lequel lesdits articles (20) sont des perles de silice poreuse recouvertes de peptides.

6. Réacteur à flux continu (10) selon la revendication 1, dans lequel le tube de chambre de réaction (14) est pourvu d'un moyen de support poreux (18) destiné aux articles discrets (20) venant garnir la chambre.

7. Réacteur à flux continu (10) selon la revendication 1, dans lequel les diamètres interne et externe dudit tube de chambre de réaction (14), de même que desdits premier tube (12) et second tube (16) de raccordement, possèdent des dimensions telles que lesdits premier tube (12) et second tube (16) de raccordement sont adaptés par ajustement serré à l'intérieur du tube de chambre de réaction (14).

8. Réacteur à flux continu (10) selon la revendication 1, dans lequel les diamètres interne et externe du premier tube de raccordement (12) ainsi que du tube de chambre de réaction (14) possèdent des dimensions telles que le premier tube de raccordement (12) est adapté par ajustement serré à l'intérieur de la chambre de réaction (14) et dans lequel les diamètres interne et externe du tube de chambre de réaction (14) ainsi que du second tube de raccordement (16) possèdent des dimensions telles que le tube de chambre de réaction (14) est adapté par ajustement serré à l'intérieur du second tube de raccordement (16) pour constituer une chambre de réaction dépourvue de volumes non balayés par le gaz.
